# EUROPÄISCHE PATENTSCHRIFT

(11) **EP 0 654 081 B1**
(45) Veröffentlichungstag und Bekanntmachung des Hinweises auf die Patenterteilung: **02.01.1997**
(21) Anmeldenummer: 93917722.6
(22) Anmeldetag: 02.08.1993
(51) Int. Cl.: C12N 15/53, C12N 9/04, C12Q 1/26

(54) **HYPOGLYCOSYLIERTE RECOMBINANTE GLUCOSEOXIDASE**
HYPOGLYCOSYLATED RECOMBINANT GLUCOSIDASE OXIDASES
OXYDASE GLUCOSIQUE RECOMBINEE HYPOGLYCOSYLEE

(30) Priorität: 07.08.1992 DE 4226095; 25.01.1993 DE 4301904
(43) Veröffentlichungstag der Anmeldung: 24.05.1995
(73) Patentinhaber: BOEHRINGER MANNHEIM GMBH, 68298 Mannheim (DE)
(72) Erfinder: KOPETZKI, Erhard, D-82377 Penzberg (DE); LEHNERT, Klaus, D-63546 Hammersbach (DE)
(86) Internationale Anmeldenummer: EP9302054
(87) Internationale Veröffentlichungsnummer: WO9403608

(56) Entgegenhaltungen:
- WO-A-89/12675
- BIOTECHNOLOGY, Bd. 9 , Juni 1991 , NEW YORK US, Seiten 559-561; DE BAETSELIER, A. ET AL: 'Fermentation of a yeast producing A. niger glucosidase oxidase: scale-up, purification and characterization of the recombinant enzyme.'

## Beschreibung

Gegenstand der Erfindung ist eine hypoglycosylierte recombinante Glucoseoxidase (GOD EC 1.1.3.4) sowie deren Herstellung und Verwendung in diagnostischen Tests.

Ein Protein kann auf 3 Arten posttranslational mit Kohlenhydraten versehen werden. Man unterscheidet zwischen:
* N-Glycosylierung
   - N-glycosidische Bindung der Kohlenhydratkette zu Asn
* O-Glycosylierung
   - O-glycosidische Bindung der Kohlenhydratkette zu Thr oder Ser
* Glycosyl-phosphatidyl-inositolanker (GPI)
   - Bestandteil einiger Membranproteine,
   - Der GPI-Anker dient zur Einlagerung in die Phospholipidmembran.

Die Glycosylierung von Proteinen ist beispielsweise beschrieben in:
- Kukuruzinska, M.A. et al., Ann. Rev. Biochem. 56 (1987) 915-944;
- Paulson, C.P., TIBS 14 (1989) 272-276;
- Warren, C.E., BFE 7 (1990) 392-395;
- Ballou, C.E., In: Strathern, J.N., et al., The Molecular Biology of the Yeast Saccharomyces, Cold Spring Harbor Laboratory, New York, pp. 355-360 (1982).
- Kornfeld, R.; Kornfeld, S., Ann. Rev. Biochem 54 (1985) 631-664;
- Tanner, W.; Lehle, L., Biochim. Biophys. Acta 906 (1987) 81-99;
- Innis, M.A., In: Barr, P.J. et al., Yeast genetic engineering, Butterworths, Stoneham, Mass, pp. 233-246 (1989).

Die O-glycosidischen Kohlenhydratstrukturen von Hefeproteinen bestehen aus einer unverzweigten Mannosekette von 1 - 5 Mannoseresten. Die O-Glycosylierung beginnt im ER (Transfer des ersten Mannoserestes) und wird im Golgi Apparat beendet.

Die N-Glycosylierung erfolgt in 2 Schritten. An einem Lipidcarrierintermediat wird eine "core" Einheit aus N-Acetylglucosamin, Mannose und Glucose aufgebaut, die im ER auf Asn-Reste von "Glycoproteinen" übertragen wird. Nach Prozessierung der Protein-gebundenen "core"-Einheit (Abspaltung der Glucosereste und eines spezifischen Mannoserestes im ER) wird im Golgi Apparat die Zuckerstruktur verlängert ("outer chain" Glycosylierung). Die Struktur der "outer chain" Glycosylierung ist Organismus spezifisch.

Die Glucoseoxidase (GOD EC 1.1.3.4) aus Aspergillus niger ist ein natürlich sezerniertes N-glycosyliertes Homodimer, mit einem Molekulargewicht von ca. 80 kDa pro Untereinheit (UE), 1 FAD als Cofaktor/UE und einer Disulfidbrücke/UE. GOD aus A. niger besitzt eine relativ einheitliche Kohlenhydratstruktur (core Glycosylierung).

Die technische Herstellung von Glucoseoxidase aus Aspergillus niger ist jedoch schwierig. Die GOD wird in Aspergillus niger offensichtlich in die Peroxisomen (Dijken, J.P. van und Veenhuis, M., Eur. J. Appl. Microbiol. 9 (1980) 275 - 283) transportiert, wodurch die Aufarbeitung erschwert wird. Unter bestimmten Bedingungen kann das Enzym aber auch ins Medium sekretiert werden (Mischak, H. et al., Appl. Microbiol. Biotech. 21 (1985) 27 - 31). Die Ausbeute an GOD ist dabei nur gering. Aus diesen Gründen wurde bereits mehrfach versucht, die Glucoseoxidase aus Aspergillus niger rekombinant in Saccharomyces cerevisiae herzustellen. Die rekombinante GOD aus Saccharomyces cerevisiae ist temperatur- und pH-stabiler als das native Enzym aus Aspergillus (De Baetselier A. et al., Biotechnology 9 (1991) 559 - 561). Bei der rekombinanten Herstellung ergaben sich zwar hohe Ausbeuten an Enzym, es zeigte sich jedoch, daß das rekombinante Enzym durch eine nichteinheitliche "outer chain Glycosylierung" von bis zu 150 Mannoseresten hinsichtlich des Kohlenhydratanteils und des Molekulargewichts von 80 bis 140 kDa/UE (UE - Untereinheit) heterogen ist. Das rekombinante Enzym ist hyperglycosyliert (Kohlenhydratanteil ca. 70% statt 16% wie beim nativen Enzym (De Baetselier et al., Biotechnology 9 (1991) 559 - 561) und besitzt deshalb ein wesentlich höheres Molekulargewicht (ca. 80 - 140 kDa/UE) als das native Enzym. Dies hat bei der Verwendung der GOD, insbesondere in diagnostischen Tests, Nachteile, z.B. besitzt das hyperglycosylierte rekombinante Enzym eine geringere spezifische Aktivität in Units pro Gewichtseinheit (ca. 65 U/mg, gegenüber 178 U/mg für das native Enzym aus Aspergillus).
- Kriechbaum, M. et al., FEBS Lett. 255 (1989) 63-66;
- Frederick K.R. et al., J. Biol. Chem. 265 (1990) 3793 - 3802;
- De Baetselier, A. et al., Biotechnology 9 (1991) 559 - 561;
- Whittington, H. et al., Curr. Genet. 18 (1990) 531 - 536;
- Rosenberg, S., WO 89/12675;

In diesen Publikationen ist auch die Sequenz der Glucoseoxidase aus Aspergillus niger beschrieben.

Die Aufgabe der vorliegenden Erfindung war es, diese Nachteile zu vermeiden und eine rekombinante Glucoseoxidase zur Verfügung zu stellen mit möglichst einheitlicher und reduzierter N-Glycosylierung (z. B. mit einem vollständigen oder partiellen Defekt in der "outer chain" Glycosylierung) und hoher spezifischer Aktivität.

Diese Aufgabe wird gelöst durch eine hypoglycosylierte rekombinante Glucoseoxidase mit einem Molekulargewicht von 68 - 80 kDa, einer spezifischen Aktivität von ca. 200 U/mg Gewichtseinheit, einem Kohlenhydratanteil von ca. 12%, erhältlich durch Expression einer das GOD-Gen aus Aspergillus enthaltenden rekombinanten DNA in den N-glycosylierungsdefekten Hefemutanten DSM 7042, DSM 7160, DSM 7338 oder DSM 7340 oder allelen Mutantenstämmen davon, Fermentation und Isolierung des Enzyms aus dem Kulturüberstand oder den Zellen.

Überraschenderweise wurde gefunden, daß aus N-glycosylierungsdefekten Hefemutanten (z.B. ngd29 Mutante) isolierte GOD wesentlich stabiler ist als das Enzym aus Aspergillus niger, bei vergleichbarem Kohlenhydratanteil, Molekulargewicht (68 - 80 kDa, vorzugsweise 68 - 75 kDa) und spezifischer Aktivität.

Die zur Herstellung der erfindungsgemäßen GOD geeigneten Hefestämme und ihre Herstellung sind beschrieben in der deutschen Patentanmeldung P 42 26 094.9, mit gleichem Zeitrang, deren Inhalt Gegenstand der Offenbarung der vorliegenden Erfindung ist.

Derartige Hefestämme sind erhältlich durch [³H]-Mannose Suizidselektion, Einführung eines oder mehrerer selektierbarer Marker (Auxotrophiebedürfnisse und/oder Resistenzen) und Auswahl derjenigen Stämme, welche nach Transformation mit dem Plasmid YEpL/GOD in das Medium nach Anzucht gemäß Standardbedingungen mehr als 10 mg/l GOD sezernieren und allel sind zu den Saccharomyces cerevisiae Mutanten ngd29 (DSM 7042, DSM 7338) oder ngd62 (DSM 7160, DSM 7340).

Das Prinzip der [³H]-Mannose Suizidselektion besteht im wesentlichen aus:
- Mutagenese (z.B. ausgehend vom Wildtypstamm X2180-1A, ATCC 26786)
- Inkubation mit [³H]-Mannose
- Anreicherung von hyperglycosylierungsdefekten Mutanten durch Lagerung der Zellen bei -80°C bis die Überlebensrate der Zellen auf 2 - 3 Zehnerpotenzen abfällt (2 - 4 Monate)
- Selektion nach Mutanten mit reduzierter N-Glycosylierung anhand von homolog exprimierter Invertase
- Analyse durch Aktivitätsanfärbung und/oder
- Immunpräzipitation von sezernierter Invertase und Bestimmung des Molekulargewichts (Glycosylierung) durch SDS-PAGE

Die Auxotrophiemarker werden durch Kreuzung der Hefestämme zu Diploiden (Isolierung der Zygoten durch Mikromanipulation) und gegebenenfalls anschließende Sporulation zu Haploiden (Tetradenanalyse) eingeführt. Der ngd-Phänotyp wurde durch Aktivitätsanfärbung von externer Invertase mittels nativer PAGE-Gele mit Saccharose und 2,3,4-Trinitrophenyletrazoliumchlorid als Substrat/Glucosereagenz ermittelt.

Die ausreichende GOD-Produktion kann bestimmt werden durch Aktivitätsbestimmung der ins Medium sezernierten GOD nach Anzucht unter Standardbedingungen. Dazu wird der zu testende Stamm (GOD Transformante) vorzugsweise nach einer selektiven Vorkulturführung in Vollmedium 3 - 4 Tage unter Schütteln inkubiert. Dem Vollmedium werden vorzugsweise Hefeextrakt, Bactopepton, Fructose und Maltose bei neutralem pH-Wert zugesetzt.

Die Bestimmung der Glucoseoxidase erfolgt beispielsweise nach dem in den Beispielen unter "allgemeine Methoden" beschriebenen Verfahren.

Erfindungsgemäße Mutanten (allele Mutanten) können durch einen Test ermittelt werden, bei dem analysiert wird, ob die zu prüfenden Mutanten in den gleichen Genen eine Mutation aufweisen wie die Hefestämme DSM 7042/7338 (ngd29) und DSM 7160/7340 (ngd62).

Dazu wird der zu prüfende Stamm jeweils mit den Hefestämmen DSM 7042/7338 und DSM 7160/7340 gekreuzt und die dabei erhaltenen diploiden Stämme analysiert.

Die zu prüfende Mutation (Stamm) ist mit den erfindungsgemäßen ngd-Mutanten (DSM 7042/7338, ngd29) und/oder (DSM 7160/7340, ngd62) allel, wenn sich die Mutationen in diploiden Zellen nicht kompensieren.

Die zu prüfende Mutation (Stamm) ist mit den erfindungsgemäßen ngd-Mutanten (DSM 7042/7338, ngd29) und/oder (DSM 7160/7340, ngd62) nicht allel, wenn sich die Mutationen in der diploiden Zelle komplementieren und ein Wildtypphänotyp hinsichtlich N-Glycosylierung resultiert.

Bevorzugte erfindungsgemäß verwendete Hefestämme sind die Stämme DSM 7042, DSM 7160, DSM 7338 und DSM 7340.

Besonders bevorzugt sind die Stämme DSM 7338 und DSM 7340.

Die Temperaturstabilität wurde an Hand von DSC-Spektren ("differential scanning calorimetry") ermittelt. Danach beträgt der Tm-Wert mindestens 73°C im Vergleich zu 68,5°C für die GOD aus Aspergillus niger.

Nach einer Temperaturbelastung bei 55°C für 2 Stunden beträgt die GOD-Restaktivität noch mindestens 30%.

Eine bevorzugte Ausführungsform der erfindungsgemäßen GOD sind C-terminale aktive GOD Fusionsproteine, welche eine Mehrzahl von zusätzlichen Histidinen oder Cysteinen enthalten. Derartige Derivate sind beispielsweise in der EP-B 0 184 355 beschrieben und sind auf einfache Weise aufzureinigen.

Ein weiterer Gegenstand der Erfindung ist ein Verfahren zur Herstellung von rekombinanter Glucoseoxidase aus Aspergillus niger in Saccharomyces cerevisiae mit einheitlicher Kohlenhydratstruktur, dadurch gekennzeichnet, daß Saccharomyces cerevisiae Stämme mit einem Defekt in dem ngd29-Gen (z.B. DSM 7042/7338) und/oder ngd62-Gen (z.B. DSM 7160/7340) mit einer rekombinanten DNA, welche das Gen für GOD enthält, transformiert werden und nach Anzucht der Zellen die Glucoseoxidase aus den Zellen oder dem Überstand gewonnen wird.

Ein weiterer Gegenstand der Erfindung ist schließlich die Verwendung einer erfindungsgemäßen Glucoseoxidase in einem diagnostischen Test.

Für Patentzwecke wurden bei der Deutschen Sammlung für Mikroorganismen und Zellkulturen GmbH (DSM), Mascheroder Weg 1 B, D-3300 Braunschweig, hinterlegt:

| | Hinterlegungsnummer | Hinterlegungsdatum |
|---|---|---|
| 1. Plasmid YEpL | DSM 7038 | 07.04.1992 |
| 2. Hefemutante BMY3-9A (ngd29) | DSM 7042 | 08.04.1992 |
| 3. " BMY3-9C (ngd29) | DSM 7193 | 24.07.1992 |
| 4. " BMY12-20D (ngd62) | DSM 7160 | 09.07.1992 |
| 5. " BMY8-12A (ngd62) | DSM 7157 | 09.07.1992 |
| 6. " BMY13-7B (mnn9) | DSM 7158 | 09.07.1992 |
| 7. " BMY13-1C (mnn9) | DSM 7159 | 09.07.1992 |
| 8. " JM 1935 | DSM 7156 | 09.07.1992 |
| 9. " DBY 746 | DSM 4316 | 14.12.1987 |
| 10. " N-BMY3-9A | DSM 7338 | 08.12.1992 |
| 11. " N-BMY13-1C | DSM 7339 | 08.12.1992 |
| 12. " N-BMY12-20D | DSM 7340 | 08.12.1992 |
| 13. " N-BMY3-9C | DSM 7341 | 08.12.1992 |

Die nachfolgenden Beispiele erläutern die Erfindung weiter.

### Beispiele

### Allgemeine Methoden

### Rekombinante DNA-Technik

Zur Manipulation von DNA wurden Standardmethoden benutzt, wie sie bei Maniatis, T. et al., In: Molecular cloning: A laboratory manual. Cold Spring Harbor Laboratory Press, Cold Spring Harbor, New York, (1989), beschrieben sind. Die verwendeten molekularbiologischen Reagenzien wurden nach den Angaben des Herstellers eingesetzt.

### Hefetransformation

Saccharomyces cerevisiae Stämme wurden entsprechend der Methode von Beggs, J.D. (Nature 275 (1978) 104-109); Ito, H. et al., J. Bacteriol. 153 (1983) 163-168 oder Delorme, E. (Applied and Environmental Microbiology 55 (1989) 2242-2246) transformiert. Als C-Quelle wurde Fructose anstelle von Glucose verwendet.

### Bestimmung der Glucoseoxidase Aktivität

Die GOD Aktivitätsbestimmung wurde in einem Volumen von 1 ml in mit Sauerstoff gesättigtem 0,1 mol/l Kaliumphosphatpuffer, pH 7,0, mit 0,18 mol/l Glucose, 15 Units/ml Meerrettich Peroxidase und 1,75 mmol/l ABTS® Glucosereagenz bei 25°C durchgeführt. Die Reaktion wurde durch Zugabe von Glucoseoxidase (10 µl GOD-haltige Probe verdünnt auf 5 - 20 mU/ml) gestartet und die Absorptionsänderung/min (ΔA/min) bei 405 nm (ε ₄₀₅ = 36,8 [mmol⁻¹ x 1 x cm⁻¹]) bestimmt. 1 Unit (U) GOD Aktivität ist definiert als die Menge Enzym, die 1 µmol Glucose pro min bei 25°C oxidiert. Die spezifische Aktivität der gereinigten A. niger GOD beträgt unter diesen Testbedingungen ca. 230 U/mg Protein.

Proteinbestimnungen Die Proteinbestimmung wurde nach der Microbiuret-Methode (Zamenhof, S. et al., Methods Enzymol. 3 (1957) 696-704) mit Rinderserumalbumin als Standard durchgeführt.

Die Proteinkonzentration gereinigter GOD Enzyme wurde anhand der optischen Dichte bei 280 nm ermittelt (1 OD₂₈₀ ≙ 1,5 mg/ml gereinigter GOD).

### Zell-Lyse und Rohextraktgewinnunq

Die Zellen aus 5 ml Anzuchtmedium (ca. 0,1 - 0,2 g Hefe, Naßgewicht) wurden abzentrifugiert. Das Zellpellet wurde einmal mit 10 mmol/l Phosphatpuffer, pH 7,0, gewaschen und anschließend mit Glasperlen durch Homogenisieren mit einem Whirlmix aufgeschloseen (Ciriacy, M., Mut. Res. 29 (1975) 315-326). Danach wurden die Zellen in 2 ml 10 mmol/l Phosphatpuffer, pH 7,0, resuspendiert/extrahiert, die Zelltrümmer abzentrifugiert und der Überstand als Rohextrakt weiterverarbeitet.

### SDS-Polyacrylamid-Gelelektrophorese (SDS-PAGE)

Lösliche Proben (Mediumüberstände und Zell-Lysate) wurden mit 1/5 Volumen 5xSDS-Probenpuffer (1xSDS-Probenpuffer: 50 mmol/l Tris-HCl, pH 6,8, 1% SDS, 1% Mercaptoethanol, 10% Glycerin, 0,001% Bromphenolblau) versetzt und 5 min bei 95°C inkubiert. Nicht lösliche Proteine der Zelltrümmerfraktion wurden mit 2 ml 1xSDS-Probenpuffer und 6 - 8 mol/l Harnstoff extrahiert, durch 5 minütiges Erhitzen auf 95°C denaturiert und durch Zentrifugation von den unlöslichen Bestandteilen abgetrennt. Danach wurden die Proteine durch SDS-PAGE aufgetrennt (Laemmli, U.K., Nature 227 (1970) 680-685) und mit Coomassie Brilliant Blue R Farbstoff angefärbt.

### Beispiel 1

### Konstruktion von Plasmiden zur Sekretion der A. niger GOD in S. cerevisiae

### Konstruktion des Hefeexpressionsvektors YEpL (Basisvektor)

Das Plasmid YEpL basiert auf dem αGlucosidase Vektor YEp/5C6b3 (Kopetzki et al., Yeast 5 (1989) 11-24; Kopetzki, et al., EP-A 0 323 838). Zur Replikation des Plasmids in E. coli dient das ca. 2,3 kBp lange EcoRI/PvuII-Fragment aus dem Plasmid pBR322 (Plasmidursprung, Ampicillinresistenzgen). Zur Replikation in Hefe enthält der Vektor das ca. 2,2 kBp lange EcoRI-Fragment aus der 2µm DNA der Hefe (subkloniert aus dem E. coli/Hefe-Shuttlevektor YEp24). Weiterhin enthält der Vektor das URA3 und LEU2d Gen zur Selektion des Plasmides in auxotrophen Hefestämmen und eine aGlucosidase-Expressionskassette (GLUCPI-Gen). Sie besteht aus dem αGlucosidasepromotor, einem Polylinker (Klonierungstelle für zu exprimierende Gene) und dem aGlucosidaseterminator. Zudem ist das MAL2-8cp Gen vorhanden, dessen Genprodukt, das MAL2-8cp-Protein, den aGlucosidasepromotor aktiviert. Der αGlucosidasepromotor ist in Gegenwart von Glucose reprimiert. Er dereprimiert nach Verbrauch der Glucose und erreicht seine maximale Aktivität nach Induktion mit Maltose.

### 1.1 Konstruktion des Plasmids YEp/KL6b3

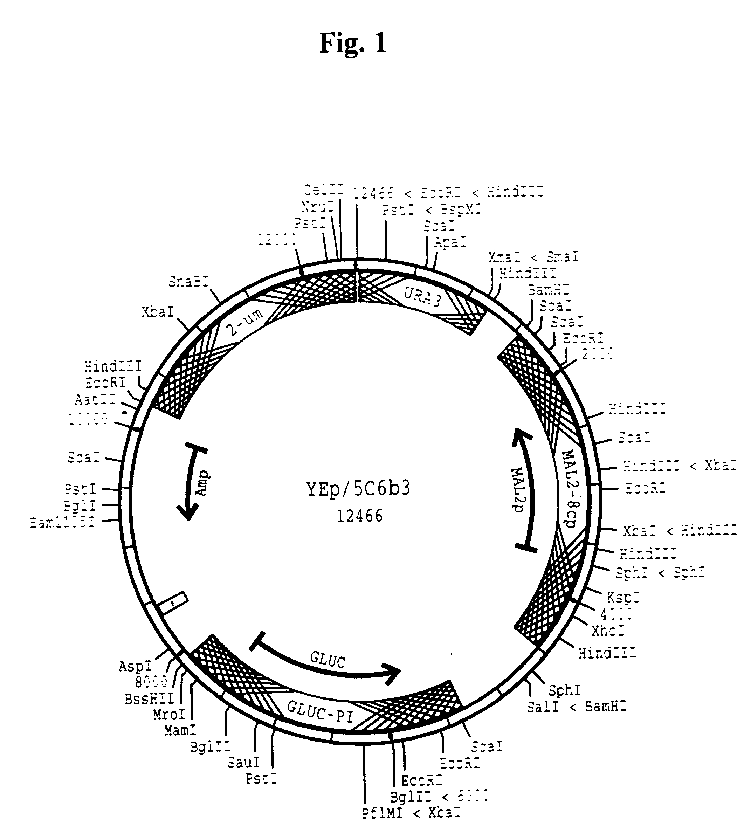

Die nicht benötigte ca. 1,4 kBp lange DNA Sequenz zwischen dem αGlucosidaseterminator und dem MAL2-8cp Promotor wurde aus dem Plasmid YEp/5C6b3 (Fig. 1) deletiert.

Dazu wurde das Plasmid YEp/5C6b3 mit XhoI linearisiert, die 5'-überhängenden Enden mit Klenow Polymerase aufgefüllt, das Plasmid mit MroI nachgeschnitten und das 8,7 kBp lange MroI/XhoI(blunt)-Vektorfragment isoliert. In einem zweiten Ansatz wurde das Plasmid YEp/5C6b3 mit den Restriktionsendonukleasen MroI und ScaI verdaut, das αGlucosidasegen enthaltende 2,5 kBp lange MroI/ScaI-Fragment isoliert und mit dem 8,7 kBp langen MroI/XhoI(blunt)-Vektorfragment ligiert. Das gewünschte Plasmid wurde durch Restriktionskartierung identifiziert und mit YEp/KL-6b3 bezeichnet.

### 1.2 Konstruktion des Plasmids YEp/KL-6b3M

In die SspI Restriktionsendonukleaseschnittstelle der 5'-nichttranslatierten Region des MAL2-8cp Gens wurde ein MluI-Linker (5'-GACGCGTC-3') ligiert. Plasmidkonstruktion: YEp/KL-6b3M.

### 1.3 Konstruktion des Plasmids YEg/KL-6b3M-MCS

Durch "polymerase chain reaktion" (PCR)-Technik (Mullis, K.B. und Faloona, F.A., Methods in Enzymol. 155 (1987) 335-350) wurde das Strukturgen der aGlucosidase entfernt und durch einen DNA-Linker ("multi cloning site", MCS) ersetzt.

Dazu wurde die GLUCPI-Promotorsequenz aus dem Plasmid YEp/KL-6b3M mittels PCR unter Verwendung des Primerpaares (siehe SEQ ID NO. 1 und SEQ ID NO. 2) amplifiziert und das ca. 410 Bp lange PCR Produkt nach Agarosegelelektrophorese isoliert.

In einer zweiten PCR Reaktion wurde die GLUCPI-Terminatorsequenz aus dem Plasmid YEp/KL-6b3M unter Verwendung des Primerpaares (siehe SEQ ID NO. 3 und SEQ ID NO. 4) amplifiziert und das ca. 860 Bp lange PCR Produkt nach Agarosegelelektrophorese isoliert.

Danach wurden äquimolare Mengen (jeweils ca. 50 pg) der isolierten PCR-Fragmente in PCR Reaktionsmix vereinigt, 5 min bei 95°C zur Denaturierung der ds-DNA inkubiert, das Reaktionsgemisch auf 60°C zum "Annealing" der komplementären MCS-haltigen singulären DNA Stränge abgekühlt, die Hybridisierungsprodukte mit Taq-Polymerase in ds-DNA überführt und in einer dritten PCR Reaktion unter Verwendung des Primerpaares (siehe SEQ ID NO. 1 und SEQ ID NO. 4) amplifiziert. Danach wurde das ca. 1,27 kBp lange PCR Produkt mit den Restriktionsendonukleasen MroI und MluI verdaut, das ca. 0,92 kBp lange MroI/MluI-GLUPI-Promotor/MCS/GLUCPI-Terminatorfragment nach Agarosegelelektrophorese isoliert und in das ca. 8,55 kBp lange MroI/MluI-YEp/KL-6b3M Vektorfragment ligiert. Das gewünschte Plasmid YEp/KL-6b3M-MCS wurde mittels Restriktionskartierung identifiziert und die mittels PCR synthetisierten DNA-Bereiche durch DNA Sequenzierung überprüft.

### 1.4 Konstruktion des Plasmids YEpL

In der folgenden Plasmidkonstruktion wurde das LEU2d Gen in das Plasmid YEp/KL-6b3M-MCS insertiert. Dazu wurde das Plasmid YEp/KL-6B3M-MCS mit CelII und SnaBI verdaut und das 8,4 kBp lange CelII/SnaBI-YEp/KL-6b3M-MCS Vektorfragment isoliert. Das LEU2d Gen wurde als ca. 2,32. kBp langes CelII/SnaBI-Fragment aus dem Plasmid pADH040-2 (Erhart, E. und Hollenberg, C.P., J.

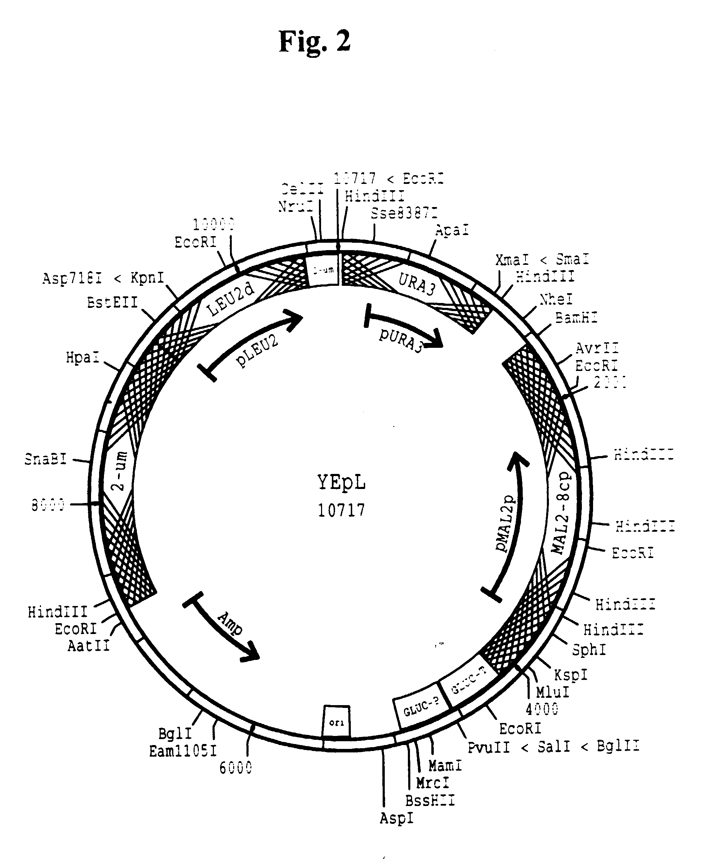

Bacteriol. 156 (1983) 625-635) isoliert und mit dem 8,4 kBp langen CelII/SnaBI-YEp/KL-6b3M-MCS Vektorfragment ligiert. Die gewünschte Plasmidkonstruktion YEpL (DSM 7038) wurde durch Restriktionskartierung identifiziert (Fig. 2).

### 1.5 Konstruktion des Plasmids YEpL/GOD

Die Klonierung des verwendeten Glucoseoxidase Gens (Stamm: NRRL-3, ATTC 9029), die Subklonierung in den pBluescript SK(+) Vektor, die DNA Sequenzierung und die Ableitung der GOD Proteinsequenz sind in der Publikation von Kriechbaum, M. et al. (FEBS Lett. 255 (1989) 63-66) beschrieben. Das GOD Gen wurde in 2 Teilbereichen (SalI Restriktionsfragmente) in pBluescript SK(+) kloniert.

Das Plasmid pSK/GOD-1.8 enthält ein ca. 1,8 kBp langes SalI-Fragment, das für die 5'- nichttranslatierte Region und den N-terminalen Bereich des GOD Strukturgens bis zur SalI-Schnittstelle an Bp-Position 164 kodiert (Bp-Position entsprechend der Numerierung bei Kriechbaum, M. et al.). Das Plasmid pSK/GOD-2.0 enthält ein ca. 2,0 kBp langes SalI-Fragment, das für den Rest des GOD Strukturgens von Bp-Position 165 bis 1853 sowie die 3'- nichttranslatierte Region stromabwärts des GOD Strukturgens kodiert.

Mittels PCR Technik wurde die 5'- und 3'- nichttranslatierte Region des GOD Gens entfernt, das GOD Strukturgen an beiden Enden mit singulären Restriktionsendonukleaseschnittstellen versehen (BglII bzw. PvuII) und zudem in der C-terminalen kodierenden Region des GOD Strukturgens eine singuläre SphI und NheI Schnittstelle eingeführt unter Erhalt einer für das native GOD Protein kodierenden DNA-Sequenz. Danach wurde das GOD Strukturgen in einer Dreifragmentligation aus den beiden PCR Fragmenten zusammengesetzt und in den Vektor YEpL inseriert. Zur Amplifikation des N-terminalen GOD Strukturgens wurde das folgende Primerpaar verwendet (siehe SEQ ID NO. 5 und SEQ ID NO. 6) und Plasmid pSK/GOD-1.8 als Template DNA.

Zur Amplifikation des restlichen GOD Strukturgens wurde das folgende Primerpaar verwendet (siehe SEQ ID NO. 7 und SEQ ID NO. 8) und Plasmid pSK/GOD-2.0 als Template DNA.

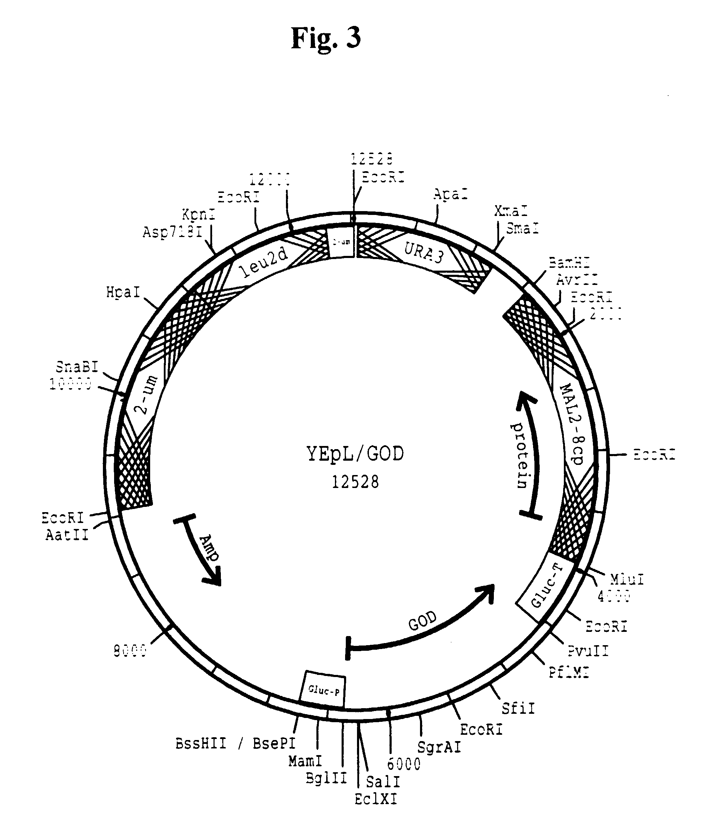

Das ca. 220 Bp lange PCR Produkt der ersten Reaktion wurde mit BglII und SalI nachgespalten und das ca. 130 Bp lange BglII/SalI-Fragment isoliert. Das ca. 2,05 kBp lange PCR Produkt der zweiten Reaktion wurde mit SalI und PvuII verdaut das ca. 1,7 kBp lange DNA Fragment isoliert. Danach wurden die PCR Fragmente in das ca. 10,7 kBp lange BglII/PvuII-YEpL Vektorfragment ligiert (Dreifragmentligation). Das gewünschte Plasmid YEpL/GOD (Fig. 3) wurde durch Restriktionskartierung identifiziert und partiell sequenziert (Klonierungsübergänge).

### 1.6 Konstruktion des Plasmids YEpL/GOD-(His)₄

Das Plasmid enthält ein modifiziertes GOD Gen, das für eine GOD Enzymvariante kodiert, die C-terminal zusätzlich 4 Histidinreste besitzt. YEpL/GOD-(His)₄ wurde aus dem Plasmid YEpL/GOD hergestellt.

Dazu wurde das Plasmid YEpL/GOD partiell mit SphI und vollständig mit PvuII gespalten, das ca. 10,7 kBp lange SphI/PvuII-Fragment isoliert und mit dem folgenden, aus 2 Oligonukleotiden (siehe SEQ ID NO. 9 und SEQ ID NO. 10) durch Hybridisierung hergestellten, DNA Linker ligiert.

Das gewünschte Plasmid YEpL/GOD-(His)₄ wurde durch Koloniehybridisierung mit radioaktiv markiertem Primer 10 als Sonde idendifiziert und durch Restriktionskartierung und partielle Sequenzierung (C-terminale Bereich des GOD Strukturgens) weiter analysiert.

### Beispiel 2

### Isolierung von Hefewirtsstämmen mit defekter N-Glycosylierung

### 2.1 [³H]-Mannose Suizidmutagenese

### Prinzip:

- Mutagenese (Ausgangsstamm: X2180-1A, Genotyp: a SUC2 mal mel gal2 CUP1; ATCC 26786)
- Inkubation mit [³H]-Mannose
- Anreicherung von hyperglycosylierungsdefekten Mutanten durch Lagerung der Zellen bei -80°C bis die Überlebensrate der Zellen auf 2 - 3 Zehnerpotenzen abfällt (2 - 4 Monate)

Ein Hefestamm wie z.B. X2180-1A (ATCC 26786) wird in YEPD-Medium (2% Bacto Pepton, 1% Hefeextrakt, Difco, und 4% Glucose) angezogen, in der logarithmischen Wachstumsphase (davon ca. 5 x 10⁸ Zellen) geerntet, mit 0,1 mol/l Natriumphosphat, pH 7, gewaschen und in 1,5 ml 0,1 mol/l Natriumphosphat, pH 7, resuspendiert. Die Zellen werden mutagenisiert durch Zusatz von 0,1 ml Ethylmethansulfonat für eine Stunde bei 25°C. 0,2 ml der so behandelten Zellen werden mit 10 ml Natriumthiosulfat (5% w/v) für 10 Minuten inkubiert, 3 x mit 0,1 mol/l Natriumphosphat, pH 7,0, gewaschen und in YEPD-Medium (2% Bacto Pepton, 1% Hefeextrakt, Difco) mit 4% Glucose resuspendiert.

Die Zellen werden bei 28°C unter Schütteln inkubiert bis eine OD von 0,6 bei 578 nm erreicht ist. 10⁶ Zellen werden mit YEP Medium (2% Bacto Pepton, 1% Hefeextrakt, Difco) gewaschen und in 0,1 ml YEP mit 0,1% Glucose resuspendiert. 2 mCi [³H]-Mannose (spezifische Aktivität 18,5 Ci/mmol) werden zugegeben und die Kultur bei 28°C für 60 Minuten inkubiert. Die Zellen werden abzentrifugiert, mit Wasser gewaschen und in YEPD, welches 25% Glyzerin enthält, resuspendiert und bei - 70°C zur Einwirkung der Radioaktivität gelagert. Nach ca. 45 - 50 Tagen, wenn die Überlebensrate der Zellen auf 1,5 - 0,2% abgefallen ist, werden Aliquote der Zellen auf YEP Agarplatten mit 2% Mannose ausplattiert und bei 30°C inkubiert.

### 2.2 Isolierung von Mutanten mit reduzierter N-Glycosylierung

Mutanten mit einem Defekt in der Proteinglycosylierung werden zunächst auf ihre Fähigkeit selektioniert [³H]-Mannose nicht einzubauen und [³⁵S]-Methionin einzubauen. Dazu läßt man die Zellen auf YEPD-Agar-Platten wachsen, repliziert die Hefekolonien auf 2 Rotband Filter (Schleicher & Schüll, Dassel, Deutschland) und inkubiert die Filter nochmals auf YEPD-Platten für 6 Stunden. Ein Filter wird dann in einer Lösung aus YEPD (eine zur Benetzung des Filters gerade ausreichende Menge), welche 0,01 mCi/ml [³⁵S]-Methionin enthält, inkubiert. Der andere Filter wird mit YEP, welches 0,2 mCi/ml [³H]-Mannose enthält, getränkt und 30 Minuten inkubiert. Die Zellen/Kolonien werden mit 5% Trichloressigsäure auf dem Filter fixiert, mit Wasser und Aceton gewaschen und durch Autoradiographie analysiert.

### 2.3 Charakterisierung der positiven Klone durch native Gelelektrophorese von externer Invertase

Das SUC2 Gen aus S. cerevisiae kodiert für 2 unterschiedlich regulierte und kompartimentierte Invertase Formen, i) eine glycosylierte vorwiegend ins Periplasma sezernierte Invertase und ii) eine intrazelluläre etwas verkürzte nicht glycosylierte Form (Carlson, M. et al., Mol. Cell. Biol. 3 (1983) 439-447). Die Invertase enthält 14 potentielle N-Glycosylierungsstellen, von denen im Durchschnitt 9 - 10 bei der sezernierten Form pro Invertase Untereinheit glycosyliert werden. Externe Wildtyp Invertase wandert durch nicht einheitliche "outer chain" Glycosylierung als diffuse Bande in nativen Gelen. Die cytoplasmatische, nicht glycosylierte Form ergibt im Gegensatz dazu eine scharfe Bande nach Aktivitätsanfärbung. Eine veränderte N-Glycosylierung läßt sich somit über die Wanderungsgeschwindigkeit und die Bandenschärfe von externer Invertase in nativen Gelen grob analysieren.

Die Hefestämme (X2180-1A Wildtypstamm und positiven Klone) wurden in 5 ml YEPS-Medium (1% Hefeextrakt, 2% Bactopepton, Difco, und 2% Saccharose) über Nacht angezogen, die Zellen in der spätlogarithmischen Wachstumsphase geerntet, einmal mit 20 mmol/l Natriumazid gewaschen und mit Glasperlen durch Homogenisieren auf einem Whirlmix aufgeschlossen. Die Zellysat-Herstellung, die native Gelelektrophorese und Aktivitätsanfärbung von Invertase mit Saccharose und 2,3,4-Trinitrophenyltetrazoliumchlorid als Substrat/Glucosereagenz wurde entsprechend der Methode von Ballou C.E. (Methods Enzymol. 185 (1990) 440-470) durchgeführt.

Die positiven Klone lassen sich anhand der Invertase Aktivitätsanfärbung in 4 Klassen einteilen:
1. Mutanten mit Wildtyp Invertase Mobilität.
2. Mutanten, die weder nichtglycosylierte noch glycosylierte Invertase synthetisieren.
3. Mutanten mit Defekten in der "outer chain" Glycosylierung (distinktes oligomeres Bandenmuster aus 3 - 4 Banden).
4. Mutanten, die zu einer ausgeprägten Unterglycosylierung von Invertase führen (größere Mobilität als Wildtyp Invertase)

### Ergebnis

Mutantenstämme der Klasse 4, im weiteren mit ngd29 (DSM 7042/7338) und ngd62 (DSM 7160/7340) bezeichnet (ngd für: "N-glycosylation defective"), synthetisieren im Vergleich zum Ausgangsstam X2180-1A eine "einheitlich glycosylierte" dimere externe Invertase (scharfe Bande und erhöhte Wanderungsgeschwindigkeit in nativen Gelen nach Aktivitätsanfärbung). Die ngd-Mutantenstämme waren osmotisch stabil, bei 30°C kultivierbar und aggregierten nicht während der Anzucht.

### Beispiel 3

### Konstruktion von glycosylierungsdefekten Hefewirtsstämmen zur Expression homologer und heterologer Proteine

Zur Einführung eines oder mehrerer durch Transformation komplementierbarer Auxotrophien wurden die ngd-Mutanten mit geeigneten Laborstämmen entsprechend der bei F. Sherman et al. (Methods in Yeast Genetics: A Laboratory Manual, Cold Spring Harbor Laboratory, Cold Spring Harbor, New York, (1981)) beschriebenen Methode gekreuzt und diploide Stämme durch Mikromanipulation isoliert. Anschließend wurden die diploiden Stämme sporuliert und Segreganten mit geeigneten Auxotrophien (z.B. ura3, leu2) und ngd-Mutation isoliert.

Dazu wurde die ngd29-Mutante zusammen mit dem Stamm DBY746 (MATα ura3-52 leu2-3,-112 trp1-289α his3-Δ1; (DSM 4316, äquivalent zu ATCC 44773) und die ngd62 Mutante mit dem Stamm JM1935 (MATα ura3 leu2 his4, DSM 7156) 6 Stunden bei 30°C in YEPD (1% Hefeextrakt, 2% Bactopepton, Difco, und 2% Glucose) inkubiert.
Anschließend wurden mit Hilfe eines Mikromanipulators (Modell: nach de Fonbrune der Firma Bachhofer, Reutlingen) Zygoten isoliert und in 5 ml YEPD über Nacht angezogen. Die Zellen wurden kurz abzentrifugiert, das Medium bis auf ca. 0,2 ml dekantiert und das Zellpellet im Restmedium resuspendiert. Diese Zellsuspension wurde auf eine Kaliumacetat-Platte (1% Kaliumacetat, 1,5% Agar) ausgebracht. Nach ca. 5 Tagen wurden die so erhaltenen Asci mit einer Impföse in 0,5 ml sterilem Wasser resuspendiert, 10 µl eines β-Glucuronidase/Arylsulfatase-Gemisches (Boehringer Mannheim) zugegeben und 10 min bei Zimmertemperatur inkubiert. Anschließend wurden 10 ml Wasser zugegeben, die Ascus-Suspension abzentrifugiert und der Überstand dekantiert. Unter dem Mikromanipulator wurden die Sporen mehrerer Asci isoliert und auf YEPD-Platten (YEPD mit 1,5% Agar) 3 Tage bei 30°C inkubiert. Von ausgekeimten Sporen wurde eine Stempelplatte angelegt, die Kolonien auf synthetischen Minimalmedien (0,67% Yeast Nitrogen Base ohne Aminosäuren, Difco; 2% Glucose; 1,5% Agar plus Zusätze: 20 mg/l Trp, His, Arg, Met; 30 mg/l Leu, Ile, Lys; 50 mg/l Phe; 100 mg/l Glu, Asp; 400 mg/l Val, Thr, Ser sowie 20 mg/l Adenin und Uracil; jeweils einer dieser Zusätze fehlte in den einzelnen Minimalmedien) gestempelt und 3 Tage bei 30°C inkubiert. Segreganten mit ngd29-Phänotyp, die Auxotrophien für Uracil und Leucin aufwiesen, wurden wie in Beispiel 2.2.
beschrieben analysiert/isoliert. In allen untersuchten Tetraden segregierte der ngd29-Phänotyp (ebenso wie der ngd62 Phänotyp) 2:2, was für eine einzelne Mutation in einem einzelnen nuklären Locus spricht.

Aus der hier beschriebenen Kreuzung DBY746 x ngd29 wurden die Stämme BMY3-9A und N-BMY3-9A (MATα leu2-3,-112 ura3-52 his3-Δ1 ngd29; DSM 7042 und DSM 7338) und BMY3-9C und N-BMY3-9C (MATα leu2-3,-112, ura3-52 ngd29; DSM 7193 und DSM 7341) erhalten.

Auf analoge Art und Weise wurden aus der Kreuzung JM1935 x ngd62 die Stämme BMY12-20D und N-BMyl2-20D (MATα leu2 ura3 his4 ngd62; DSM 7160 und DSM 7340) erhalten.

### Beispiel 4

### Vergleich der Expression/Sekretion von nativer A. niger GOD und der GOD-(His)₄ Variante in Wildtyp und glycosylieningsdefekten Hefewirtsstämmen

Die GOD aus A. niger ist ein natürlicherweise sezerniertes, glycosyliertes dimeres Enzym. Pro Untereineinheit sind 8 potentielle N-Gycosylierungsstellen (Sequons) und 3 Cysteinreste vorhanden, von denen zwei eine Disulfidbrücke ausbilden. In S. cerevisiae Wildtyp Stämmen exprimierte GOD wird ins Medium sezerniert und ist hinsichtlich des Molekulargewichts auf Grund einer nicht einheitlichen "outer chain" Glycosylierung (Hyperglycosylierung) sehr heterogen (Frederick, K.R. et al., J. Biol. Chem. 265 (1990) 3793-3802; De Baetselier, A. et al., Biotechnology 9 (1991) 559-561; Whittington, H. et al., Curr. Genet. 18 (1990) 531-536). Das prozessierte (Abspaltung einer 22 Aminosäuren langen Signalsequenz) A. niger GOD Protein besteht aus 583 Aminosäuren mit einem potentiellen Molekulargewicht von 63 273 Da (Frederick, K.R. et al., J. Biol. Chem. 265 (1990) 3793-3802).

Die Plasmide YEpL/GOD (Beispiel 1.5) und YEp/GOD-(His)₄ (Beispiel 1.6) wurden in den Wildtypstamm JM1935 (MATα leu2 ura3 his4 MAL4) DSM 7156 und N-BMY3-9A oder BMY3-9A (siehe Beispiel 3) transformiert und die Transformanten auf Minimalmediumagarplatten mit 1,5% Agarose, 0,67% YNB (Yeast Nitrogen Base, Salz-Vitamin-Gemisch, Difco) 0,5% CAA (Casaminosäuren, Proteinhydrolysat, Difco) und 2% Fructose als C-Quelle selektioniert (Uracil-Selektion).

### 4.1 Anzucht der GOD-Transformanten

Zur Amplifikation der Plasmidkopienzahl (Selektion auf das Plasmid-kodierte LEU2d-Allel; Beggs, J.D., Nature 275 (1978) 104-109; Erhart, E. und Hollenberg, C.P. J., Bacteriol. 156 (1983) 625-635) wurden die Transformanten auf Minimalmediumplatten ohne Leucin ausgestrichen (1,5% Agarose, 0,67% YNB , Difco, 60 mg/l Adenin und 2% Fructose).

Vorkulturanzuchten wurden in Leucin Selektivmedium mit 0,67% YNB und 4% Fructose in Schüttelkolben bei 30°C für 48 Std. durchgeführt und zum Animpfen von Expressionskulturen (Inoculum: 1 - 2%) verwendet. Die Hauptkultur (1 l Schüttelkultur) wurde bei 30°C in Vollmedium mit 2% Hefeextract, 4% Bactopepton, Difco, 0,1 mol/l Phosphatpuffer, pH 7,0, 1% Fructose und 6% Maltose für 3 - 4 Tage unter Schütteln inkubiert. Nach 48 und 72 Std. wurden Proben entnommen und das Zellwachstum (Bestimmung der optischen Dichte bei 600 nm, OD₆₀₀), die ins Medium sekretierte GOD Aktivität und die in den Zellen verbleibende GOD Aktivität nach Zell-Lyse im Rohextrakt bestimmt.

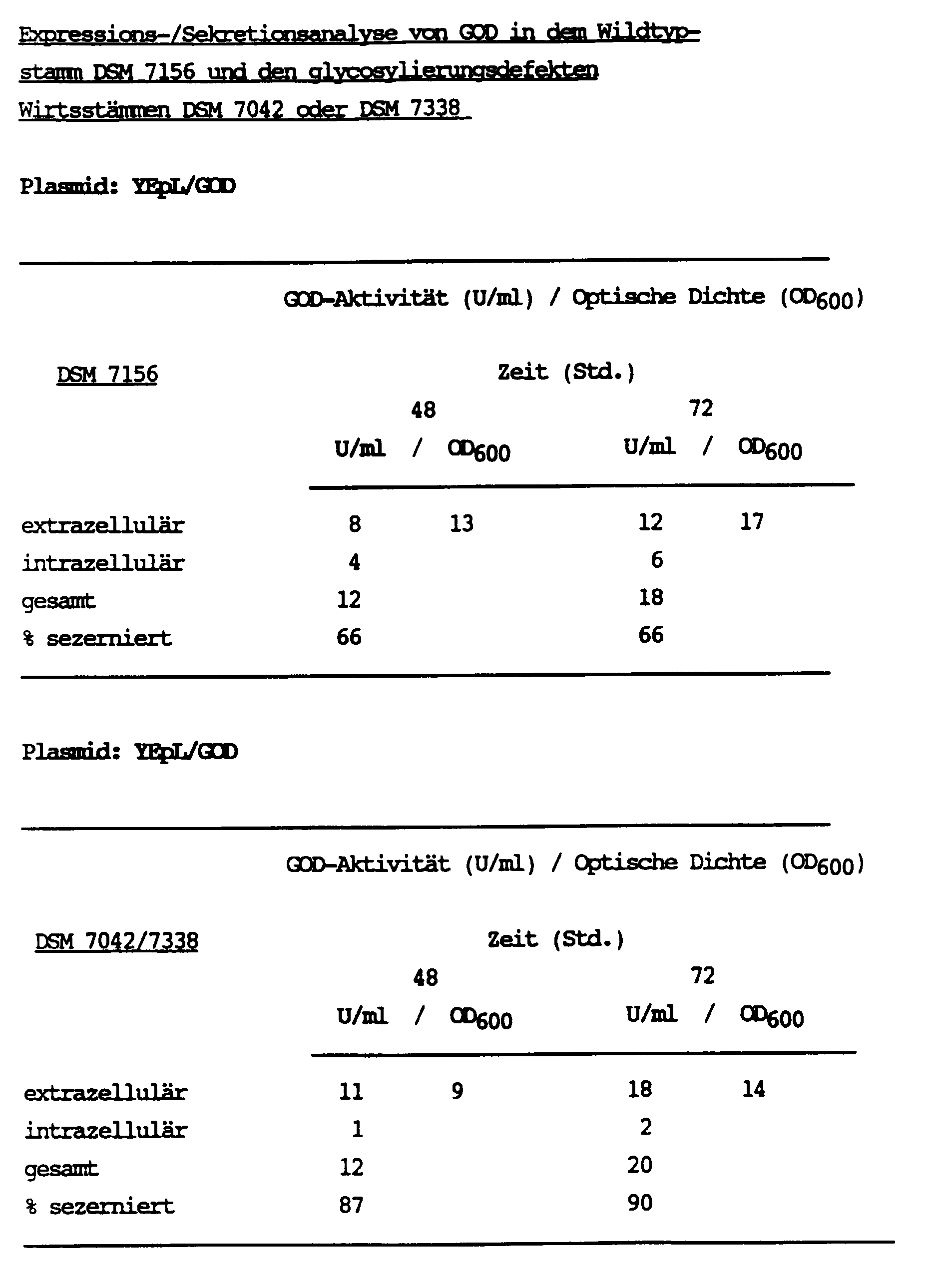

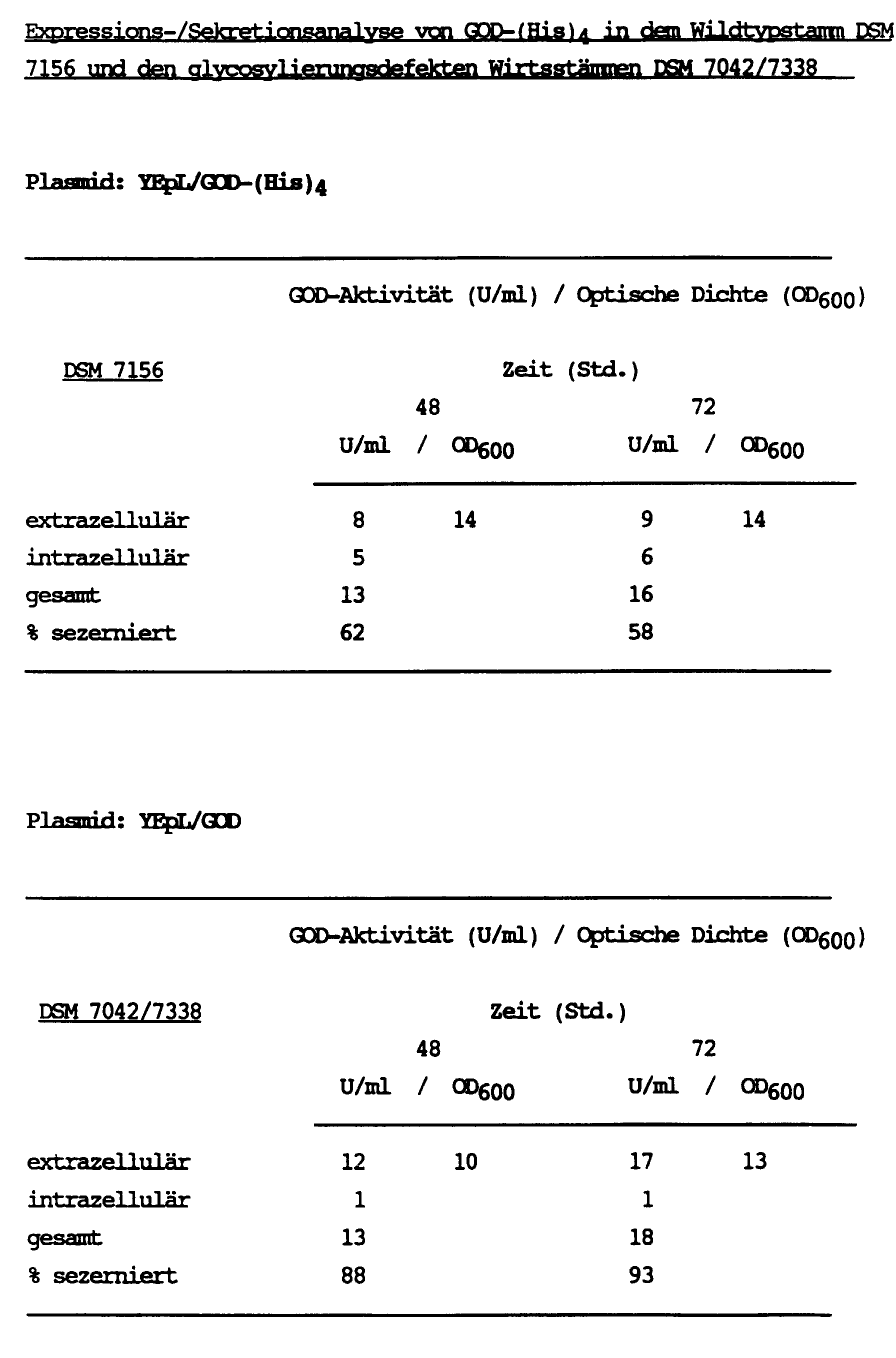

### Ergebnis

Hinsichtlich Expression und Sekretion wurden für die GOD und GOD-(His)₄ Variante keine signifikanten Unterschiede gefunden.

### 4.2 SDS-PAGE von sezernierter GOD

Das in den glycosylierungsdefekten Wirtsstämmen DSM 7042/7338 (ngd29) und DSM 7160/7340 (ngd62) exprimierte (ins Medium sezernierte) GOD-(His)₄ Enzym wurde mit dem in dem Wildtypstamm DSM 7156 exprimierten (sezernierten) Enzym und gereinigter GOD aus A. niger (Boehringer Mannheim, BRD) durch SDS-PAGE und anschließende Proteinanfärbung näher charakterisiert. Die GOD-haltigen Mediumüberstände aus dem Wildtypstamm wurden vor der Elektrophorese 10fach durch TCA-Fällung konzentriert. Kohlenhydratfreies GOD-(His)₄ Enzym wurde enzymatisch mit N-Glycosidase F hergestellt und als Größenstandard verwendet.

### Enzymatische Deglycosylierung mit N-Glycosidase F

Die Deglycosylierung wurde in Anlehnung an die von Haselbeck, A. und Hösel, W. publizierte Methode (Topics in Biochemistry 8 (1988) 1-4) durchgeführt. 0,1 ml GOD-(His)₄-haltiger Mediumüberstand wurde mit Trichloressigsäure (Endkonzentration: 10%) gefällt, die präzipitierten Proteine abzentrifugiert, das Proteinpellet mit 70% Ethanol gewaschen, im Vakuum getrocknet, in 10 µl 20 mmol/l Kaliumphosphatpuffer, pH 7,2, mit 1% SDS aufgenommen und 3 min auf 95°C erhitzt. Nach Abkühlung auf Raumtemperatur wurde die Probe mit 20 mmol/l Kaliumphosphatpuffer, pH 7,2, Octylglucosid (Endkonzentration: 0,5%) und 5 Units N-Glycosidase F auf 0,1 ml verdünnt, 1 - 12 Std. bei 37°C inkubiert und anschließend mit 25 µl 5xSDS-Puffer (siehe oben) versetzt.

### Ergebnis

Die in den glycosylierungsdefizienten ngd-Mutantenstämmen exprimierten GOD Enzyme (GOD und GOD-(His)₄) sind als dominante einheitliche Bande mit einem Molekulargewicht von ca. 80 kDa nach Proteinanfärbung in SDS-PAGE Gelen sichtbar. Dieses Experiment zeigt die fehlende "outer chain" Glycosylierung der GOD Enzyme und deutet auf eine einheitliche "core" ähnliche Glycosylierung hin. Die ngd-Mutantenstämme weisen hinsichtlich der Glycosylierung einen mnn9 ähnlichen Phänotyp auf. Im Gegensatz dazu sind die in Wildtypstämmen exprimierten GOD Enzyme nur als sehr diffuse Banden zu erkennen, die sich über einen Molekulargewichtsbereich von ca. 80 - 200 kDa erstrecken.

### Beispiel 5

### Charakterisierung der N-glycosylierungsdefekten ngd-Mutanten anhand des Wachstums auf YEPD Agarplatten mit Orthovanadat oder Hygromycin B

Glycosylierungsdefekte Mutanten wie z.B. mnn8, mnn9 und mnnl0 zeigcn eine erhöhte Orthovanadat Resistenz und eine erhöhte Sensitivität gegenüber dem Antibiotikum Hygromycin B. Der Resistenz-/Sensitivitätsphänotyp ermöglicht eine Unterscheidung/Gruppierung von N-glycosylierungsdefekten Mutanten (Ballou, L. et al., Proc. Natl. Acad. Sci. 88 (1991) 3209-3212).

Die zu untersuchenden Stämme wurden in YEPD Medium (5 ml Rollerkultur) über Nacht angezogen und die Stämme/Kulturen mit YEPD Medium auf eine optische Dichte (OD₆₀₀) von exakt 0,05 eingestellt. Danach wurden jeweils 20 µl Zellsuspension auf YEPD-Agarplatten mit 2 - 15 mmol/l Natriumorthovanadat oder 10 - 200 µg/ml Hygromycin B gespottet. Nach 2 Tagen Inkubation bei 30°C wurde das Wachstum der Zellflecken ausgewertet (siehe Tabelle).

| Wachstumsphänotyp von Hefezellen auf YEPD Agarplatten mit Natriumorthovanadat oder Hygromycin B | | | | | | | | | | | | |
|---|---|---|---|---|---|---|---|---|---|---|---|---|
| Stamm | Orthovanadat Resistenz mmol/l | | | | | | | | Hygromycin Resistenz (µg/ml) | | | |
| | 2 | 3 | 4 | 5 | 6 | 7 | 10 | 15 | 10 | 50 | 100 | 200 |
| DBY 746 (Wildtyp) | + | + | + | + | - | - | - | - | + | + | - | - |
| X2180-1A (Wildtyp) | + | + | + | + | - | - | - | - | + | + | - | - |
| LB347-1C (mnn9)¹' | + | + | + | + | + | + | + | + | - | - | - | - |
| BMY3-9A (ngd29) | + | + | + | + | + | - | - | - | + | ± | - | - |
| BMY12-20D (ngd62) | + | + | + | ± | - | - | - | - | + | ± | - | - |
| N-BMY3-9A (ngd29) | + | + | + | + | + | - | - | - | + | ± | - | - |
| N-BMY12-20D (ngd62) | + | + | + | ± | - | - | - | - | + | ± | - | - |
| + Wachstum ± sehr langsames Wachstum - kein Wachstum | | | | | | | | | | | | |

| | | | | | | | | | | | | |
|---|---|---|---|---|---|---|---|---|---|---|---|---|
| ¹⁾ J. Biol. Chem. 259 (1984) 3805-3811 | | | | | | | | | | | | |

### Ergebnis

Die ngd-Mutanten zeigen Unterschiede hinsichtlich des Resistenzmusters untereinander, zur mnn9 Mutante und zu Wildtypstämmen.

### Beispiel 6

### Charakterisierung/Identifizierung der ngd-Mutanten (Allelietest)

Ein Allelietest dient zur Identifizierung (Unterscheidung) von Genen und Gendefekten (Mutationen). Mit dieser Technik läßt sich analysieren, ob 2 Mutanten allel sind (im gleichen Gen eine Mutation aufweisen). Die ngd-Mutanten wurden untereinander und zur mnn9 Mutante auf Allelie untersucht.

Die Allelieteste wurden mittels genetischer Standardtechniken durchgeführt (siehe: Sherman, F.; Fink, G.R.; Hicks, J.B., Methods in Yeast Genetics: A Laboratory Manual. Cold Spring Harbor Laboratory, Cold Spring Harbor, New York, (1981); Guthrie, C. und Fink, G.R. (eds.), Guide to Yeast Genetics and Molecular Biology, Methods Enzymol. 194 (1991)).

### Prinzip:

Zwei zu analysierende haploide Mutantenstämme unterschiedlichen Paarungstyps mit sich komplementierenden Auxotrophiebedürfnissen werden gekreuzt und die diploiden Stämme auf Platten mit Minimalmedium selektioniert. Die Diploidie der isolierten Stämme wird durch das Vorhandensein von a und α Paarungstyp spezifischen DNA Sequenzen mittels PCR Analyse entsprechend der Methode von Huxley, C. et al. (Trends Genet. 6 (1190) 236) bestätigt.

Zwei Mutanten sind allel, d.h. besitzen eine Mutation im gleichen Gen, wenn sich die Mutationen in der diploiden Zelle nicht komplementieren.

Zwei Mutanten sind nicht allel, d.h. besitzen eine Mutation in 2 unterschiedlichen Genen, wenn sich die Mutationen in der diploiden Zelle komplementieren und ein Wildtyp Phänotyp resultiert.

### Verwendete Stämme:

| | |
|---|---|
| BMY3-9C (MATα leu2-3,-112 ura3-52 ngd29) | DSM 7193 |
| BMY8-12A (MATa trp1-289^{a} his3-Δ1 ngd62) | DSM 7157 |
| BMY13-1C (MATα ura3-52 leu2-3,-112 his3-Δ1 mnn9) | DSM 7159 |
| BMY13-7B (MATa leu2-3,-112 his3-Δ1 mnn9) | DSM 7158 |
| BMY12-20D(MATα leu2 ura3 his4 ngd62) | DSM 7160 |
| N-BMY3-9C (MATα leu2-3,-112 ura3-52 ngd29) | DSM 7341 |
| N-BMY13-1C (MATα ura3-52 leu2-3,-112 his3-Δ1 mnn9) | DSM 7339 |
| BMY12-20D(MATα leu2 ura3 his4 ngd62) | DSM 7340 |

| Kreuzungspartner | | Phänotyp der Haploiden | Selektion Diploide | Phänotyp der Diploiden |
|---|---|---|---|---|
| MATα | MAta | | | |
| BMY3-9C x BMY8-12A | | ngd29xngd62 | his leu | Wildtyp |
| BMY3-9C x BMY13-7B | | ngd29xmnn9 | his ura | Wildtyp |
| BMY13-1C BMY8-12A | | mnn9xngd62 | trp ura | Wildtyp |
| BMY12-20D x BMY13-7B | | ngd62xmnn9 | his | Wildtyp |

| Kreuzungspartner | | Phänotyp der Haploiden | Selektion Diploide | Phänotyp der Diploiden |
|---|---|---|---|---|
| MATα | MAta | | | |
| N-BMY3-9C x BMY8-12A | | ngd29xngd62 | his leu | Wildtyp |
| N-BMY3-9C x BMY13-7B | | ngd29xmnn9 | his ura | Wildtyp |
| N-BMY13-1C x BMY8-12A | | mnn9xngd62 | trp ura | Wildtyp |
| N-BMY12-20D x BMY13-7B | | ngd62xmnn9 | his | Wildtyp |
| SC = synthetisches Komplettmedium (0,67% Yeast Nitrogen Base ohne Aminosäuren, Difco; 2% Glucose; 1,5% Agar plus Zusätze: 20 mg/l Trp, His, Arg, Met; 30 mg/l Leu, Ile, Lys; 50 mg/l Phe; 100 mg/l Glu, Asp; 400 mg/l Val, Thr, Ser sowie 20 mg/l Adenin und Uracil; Die Aminosäuren Ura, His und Trp wurden, wie in der Tabelle in der Spalte Selektion der Diploide, angegeben in den einzelnen Minimalmedien weggelassen. | | | | |

### Ergebnis:

Die Mutanten ngd29 und ngd62 unterscheiden sich untereinander und sind unterschiedlich zu mnn9 (nicht allel).

### Beispiel 7

### Isolierung von GOD und GOD(His)₄ aus Wildtyp und hyperglycosylierungsdefekten Hefestämmen

### 7.1 Isolierung von GOD-(His)4 mittels Metallchelatchromatographie

Mit diesem Isolierverfahren wurde die GOD Variante GOD(His)4 aus dem Kulturfiltrat von BMY3-9A/GOD-(His)4-Zellen und BMY12-20D/GOD-(His)4-Zellen (hyperglycosylierungsdefekte Wirtsstämme) isoliert.

Das Kulturfiltrat wurde mit Natronlauge auf pH 7,5 titriert und auf eine mit 10 mmol/l Kaliumphosphatpuffer, pH 7,5, äquilibrierte NTA-Säule aufgetragen (Säulenvolumen 25 ml; NTA-Gel der Firma Diagen, Düsseldorf; Hochuli, E. et al., J. Chromatography 411 (1987) 177-184; Hochuli, E. et al., Biotechnology 6 (1988) 1321-1325). Die Säule wurde mit 5 - 10 Säulenvolumen 1 mol/l Natriumchlorid in 10 mmol/l Kaliumphosphatpuffer, pH 7,5, und mit 5 - 10 Säulenvolumen 10 mmol/l Kaliumphosphatpuffer, pH 7,5, nachgewaschen. Danach wurde das GOD-(His)₄ Enzym mit 0,1 mol/l Imidazol in Äquilibrierungspuffer, pH 7,5, eluiert und die GOD-(His)₄-haltigen Fraktionen (gelb) gegen 10 mmol/l Kaliumphosphatpuffer, pH 7,5, dialysiert.

### 7.2 Isolierung von GOD und GOD Varianten durch Ionenaustauschchromatographie an O-Sepharose ff nach vorheriger Konzentrierung und Dialyse

Nach diesem Verfahren wurde native GOD und hyperglycosylierte GOD aufgereinigt.

100 ml sterilfiltriertes Kulturfiltrat wurden mit 33 g festem Ammoniumsulfat (AS-Sättigungskonzentration 55%) unter langsamen Rühren versetzt, die präzipitierten Proteine nach 1 - 2 Std. Inkubation bei Raumtemperatur abzentrifugiert, in 25 ml 25 mmol/l Kaliumphosphatpuffer, pH 7,5, gelöst und gegen den gleichen Puffer dialysiert (4 X 10 l, 24 Std., 4°C).

Danach wurde das Dialysat auf eine mit 25 mmol/l Kaliumphosphat-Puffer, pH 7,5, äquilibrierte Q-Sepharose ff Säule (Säulenvolumen 12 ml) aufgetragen und mit 5 - 10 Säulenvolumen Äquilibrierungspuffer nachgewaschen. Die gebundenen GOD Enzyme wurden durch einen Gradienten von 0 - 1 mol/l KCl in Äquilibrierungspuffer (ca. 10 Säulenvolumen) eluiert und die GOD-haltigen (gelben) Fraktionen vereinigt.

### Beispiel 8

### Biochemische Charakterisierung der isolierten GOD Enzyme

### 8.1 Bestimmung der spezifischen GOD Aktivität

Die Bestimmung der GOD Aktivität erfolgt wie in dem Abschnitt allgemeine Methoden beschrieben.

| Spezifische Aktivität von GOD und GOD-(His)4 exprimiert in A. niger, S. cerevisiae (Wildtyp) und S. cerevisiae (hyperglycosylierungsdefekte Mutanten) | | | |
|---|---|---|---|
| Enzym | Organismus/Glycosylierung | spez. Aktivität (U/mg Protein) | spez. Aktivität (U/mg Enzym) |
| GOD | (A. niger) | 225 | 195 |
| GOD | (WT) | 230 | 69 |
| GOD | (ngd29) | 228 | 196 |
| GOD | (ngd62) | 213 | 220 |
| GOD-(His)4 | (WT) | 220 | 68 |
| GOD-(His)4 | (ngd29) | 223 | 200 |
| GOD-(His)4 | (ngd62) | 230 | 225 |
| A. niger, GOD aus A. niger, Reinheit II (Boehringer Mannheim) WT, S. cerevisiae Wildtyp ngd29, S. cerevisiae hyperglycosylierungsdefekte ngd29 Mutante ngd62 S. cerevisiae hyperglycosylierungsdefekte ngd62 Mutante | | | |

### 8.2 Bestimmung des Molekulargewichts durch SDS-Polyacrylamid-Gelelektrophorese (SDS-PAGE)

Die gereinigten GOD Enzyme wurden mit 1/5 Volumen 5xSDS-Probenpuffer (1xSDS-Probenpuffer: 50 mmol/l Tris-HCl, pH 6,8, 1% SDS, 1% Mercaptoethanol, 10% Glycerin, 0,001% Bromphenolblau) versetzt und 5 min bei 95°C inkubiert. Danach wurden die Proteine durch SDS-PAGE aufgetrennt (Laemmli, U.K., Nature 227 (1970) 680-685) und mit Coomassie Brilliant Blue R Farbstoff angefärbt.

| Molekulargewicht/Untereinheit nach SDS-PAGE von GOD und GOD (His)₄ exprimiert in A. niger, S. cerevisiae (Wildtyp) und S. cerevisiae (hyperglycosylierungsdefekte Mutanten) | | |
|---|---|---|
| Enzym | Organismus/Glycosylierung | Molekulargewicht/Untereinheit (kDe) |
| GOD | (A. niger) | ca. 80 |
| GOD | (WT) | 80 - 140 |
| GOD | (ngd29) | ca. 80 |
| GOD | (ngd62) | ca. 80 |
| GOD-(His)4 | (WT) | 80 - 140 |
| GOD-(His)4 | (ngd29) | ca. 80 |
| GOD-(His)4 | (ngd62) | ca. 80 |
| A. niger, GOD aus A. niger, Reinheit II (Boehringer Mannheim) WT, S. cerevisiae Wildtyp ngd29, S. cerevisiae hyperglycosylierungsdefekte ngd29 Mutante ngd62, S. cerevisiae hyperglycosylierungsdefekte ngd62 Mutante | | |

### 8.3 Bestimmung des Kohlenhydratanteils (AnthronReaktion)

Der Kohlenhydratanteil der GOD Enzyme aus unterschiedlichen Organismen bzw. Hefestämmen wurde in Anlehnung an die Methode von Ashwell, G. (Methods Enzymol. 3 (1957) 84) bestimmt.

Hierzu werden 0,5 ml gereinigtes GOD Enzym (Konzentration 20 - 100 U/ml in H₂O) mit 5 ml Anthron-Reagenz gemischt, die Lösung 5 Minuten bei 25°C inkubiert und danach 15 Minuten im siedenden Wasserbad erhitzt. Nach Abkühlung der Probe auf 25°C wird die Extinktion bei 630 nm gegen einen Reagentienleerwert bestimmt. Mittels einer simultan erstellten Mannose-Eichkurve mit Mannose-Standardlösungen von 5, 25, 75 und 100 µg/ml Mannose wird der Kohlenhydratanteil der GOD Probe ermittelt.

### Herstellung des Anthron-Reagenzes:

66 ml konzentrierte Schwefelsäure werden vorsichtig mit 34 ml Wasser verdünnt. Nach Abkühlung auf 80°C werden 50 mg Anthron und 1 g Thioharnstoff in der Schwefelsäure gelöst. Das Anthron-Reagenz ist zwei Wochen bei 4°C haltbar.

| Kohlenhydratanteil von GOD und GOD-(His)₄ exprimiert in A. niger, S. cerevisiae (Wildtyp) und S. cerevisiae (hyperglycosylierungsdefekte Mutanten) | | |
|---|---|---|
| Enzym | Organismus/Glycosylierung | Kohlenhydratanteil (%) (bezogen auf Protein) |
| GOD | (A. niger) | 13 |
| GOD | (WT) | 71 |
| GOD | (ngd29) | 12,5 |
| GOD | (ngd62) | 13 |
| GOD-(His)4 | (WT) | 65 |
| GOD-(His)4 | (nfd29) | 11 |
| GOD-(His)4 | (ngd62) | 12 |
| A. niger, GOD aus A. niger, Reinheit II (Boehringer Mannheim) WT, S. cerevisiae Wildtyp ngd29, S. cerevisiae hyperglycosylierungsdefekte ngd29 Mutante ngd62, S. cerevisiae hyperglycosylierungsdefekte ngd62 Mutante | | |

### 8.4 Bestimmung des Km-Wertes

Der Km-Wert der unterschiedlichen GOD Enzyme wurde gemäß der Vorschrift von Michal, G., Methods of Enzymatic Analysis, Vol. 1, Bergmeyer, H.U. (ed.) Verlag Chemie Weinheim, Academic Press, New York und London, pp. 144 - 156 (1974) bestimmt.

| Km-Wert von GOD und GOD-(His)₄ exprimiert in A. niger, S. cerevisiae (Wildtyp) und S. cerevisiae (ngd29 Mutante) | | | |
|---|---|---|---|
| Enzym | Organismus/Glycosylierung | Km | [mol x 1⁻¹] |
| GOD | (A. niger) | 0,03 | |
| GOD | (WT) | 0,03 | |
| GOD | (ngd29) | 0,03 | |
| GOD-(His)4 | (WT) | 0,03 | |
| GOD-(His)4 | (ngd29) | 0,03 | |
| A. niger: GOD aus A. niger, Reinheit II (Boehringer Mannheim) WT : S. cerevisiae Wildtyp ngd29 : S. cerevisiae hyperglycosylierungsdefekte ngd29 Mutante | | | |

### 8.6 Ermittlung der Temperaturstabilität von GOD und GOD-(His)₄ exprimiert in A. niger, S. cerevisiae (Wildtyp) und S. cerevisiae (ngd29 Mutante)

Die Temperaturstabilität der unterschiedlichen GOD-Enzyme wurde durch "differential scanning calorimetry" (DSC) ermittelt. Dazu wurde der Denaturierungspunkt (Tm) der GOD Enzyme in einem definierten Lösungsmittel (H₂O), bei definierter GOD Proteinkonzentration (20 mg/ml) und definierter Aufheizrate (10°C/min) bestimmt (siehe Tabelle).

| Tm-Werte (aus DSC-Spektren) von GOD und GOD-(His)₄ exprimiert in A. niger und S. cerevisiae (ngd29) Mutante) | | |
|---|---|---|
| Enzym | Organismus/Glycosylierung | "differential scanning calorimetry" Tm-Wert (°C) |
| GOD | (A. niger) | 68,5 |
| GOD | (ngd29) | 74,7 |
| GOD-(His)4 | (ngd29) | 75,8 |
| A. niger, GOD aus A. niger, Reinheit II (Boehringer Mannheim) ngd29, S. cerevisiae hyperglycosylierungsdefekte ngd29 Mutante | | |

### GOD Restaktivität nach Temperaturbelaetung bei 55°C von GOD und GOD-(His)₄ exprimiert in A. niger und S. cerevisiae (ngd29 Mutante)

Zur Ermittlung der Temperaturstabilität wurden die unterschiedlichen GOD-Enzyme in einer Konzentration von 25 U/ml in 0,2 mol/l Natriumphosphatpuffer, pH 7,5, bei 55°C inkubiert und nach 2 Stunden die GOD Restaktivität, wie in dem Abschnitt allgemeine Methoden beschrieben, bestimmt.

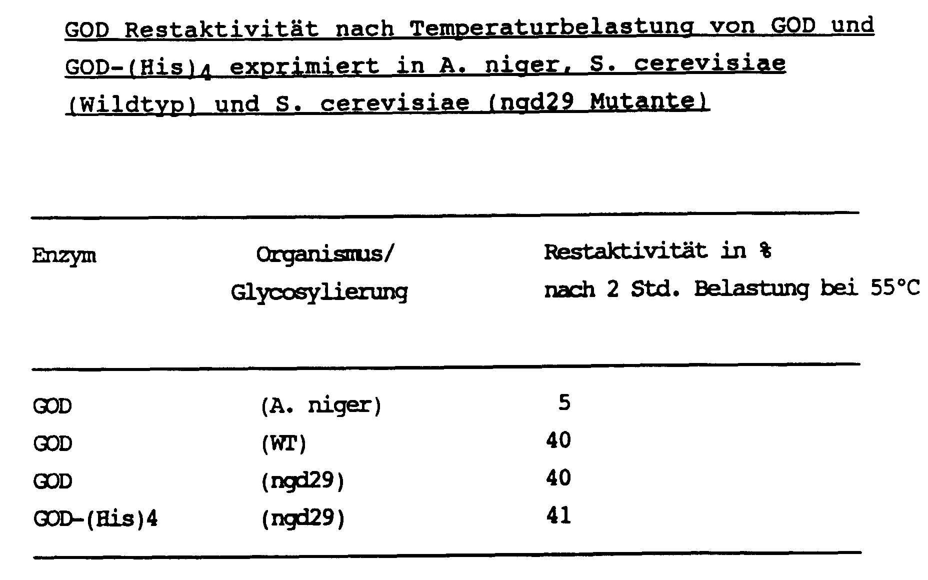

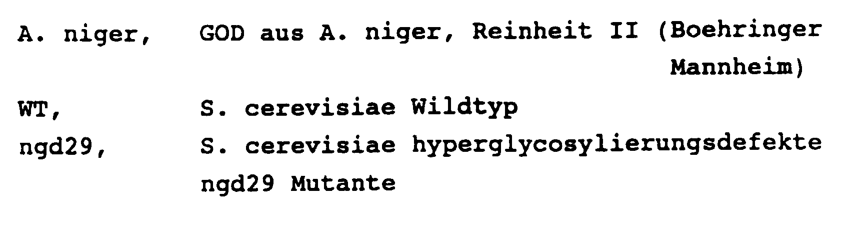

### 8.7 Ermittlung der pH-Stabilität von GOD und GOD-(His)₄ exprimiert in A. niger und S. cerevisiae (ngd29 Mutante)

Die pH-Stabilität der unterschiedlichen GOD-Enzyme wird mittels DSC ermittelt. Dazu wurde der Denaturierungspunkt (Tm) der GOD Enzyme in einem definierten Lösungsmittel (100 mmol/l Citratpuffer für pH-Werte von 3,5 - 6,5 und 100 mmol/l Boratpuffer für pH-Werte von 7,5 - 9,5 bei definierter GOD Proteinkonzentration (25 mg/ml) und definierter Aufheizrate (10°C/Minute) in Abhängigkeit vom pH-Wert ermittelt.

### Ergebnis:

GOD und GOD-(His)₄ isoliert aus der hyperglycosylierungsdefekten ngd29-Mutante verhält sich hinsichtlich pH-Stabilität wie native Aspergillus niger GOD.

### Publikationen:

Bekkers, A.C.A.P.A.; Franken, P.A.F.; Van den Bergh, C.J.; Verbakel, J.M.A.; Verheij, H.M.; De Haas,G.H.: The use of genetic engineering to obtain efficient production of porcine pancreatic phospholipase A2 by Saccharomyces cerevisiae. Biochim. Biophys. Acta 1089, 345-351 (1991).

Ballou, L.; Cohen, R.E.; Ballou,C.E.: Saccharomyces cerevisiae mutants that make mannoproteins with a truncated carbohydrate outer chain. J. Biol. Chem. 255, 5986-5991 (1980).

Ballou, C.E.: Yeast cell wall and cell surface. In: Strathern, J.N.; Jones, E.W.; Broach, J.R. (eds.), The Molecular Biology of the Yeast Saccharomyces, Metabolism and Gene Expression, Cold Spring Harbor Laboratory, New York, pp. 335-360 (1982).

Ballou, L.; Alvarado, E.; Tsai, P.; Dell, A; Ballou, C.E.: Protein glycosylation defects in the Saccharomyces cerevisiae mnn7 mutant class. J. Biol. Chem. 264, 11857-11864 (1989).

Ballou, C.E.: Isolation characterization, and properties of Saccharomyces cerevisiae mmn mutants with nonconditional protein glycosylation defects. Methods Enzymol. 185, 440-470 (1990).

Ballou, L.; Hitzeman, R.A.; Lewis, M.S.; Ballou, C.S.: Vanadate-resistant yeast mutants are defective in protein glycosylation. Proc. Natl. Acad. Sci. 88, 3209-3212 (1991).

Beggs, J.D.: Transformation of yeast by a replicating hybrid plasmid. Nature 275, 104-109 (1978).

Carlson, M.; Taussig, R.; Kustu, S.; Botstein, D.: The secreted form of invertase in Saccharomyces cerevisiae is synthesized from mRNA encoding a signal sequence. Mol. Cell. Biol. 3, 439-447 (1983).

Ciriacy, M.: Genetics of alcohol dehydrogenase in Saccharomyces cerevisiae. I. Isolation and genetic analysis of adh-mutants. Mut. Res. 29, 315-326 (1975). De Baetselier, A.; Vasavada, A.; Dohet, P.; Ha-Ti, V.; De Beukelaer, M.; Erpicum, T.; De Clerk, L.; Hanotier, J.; Rosenberg, S.: Fermentation of yeast producing A. niger glucose oxidase: scale up,
purification and characterization of the recombinant enzyme. Biotechnology 9, 559-561 (1991).

Delorme, E.: Transformation of Saccharomyces cerevisiae by electroporation. Applied and Environmental Microbiology 55, 2242- 2246 (1989).

Dijken, J.P. van; Veenhuis M.: Cytochemical localization of glucose oxidase in peroxisomes of Aspergillus niger. European J. Appl. Microbiol. Biotechnol. 9, 275-283 (1980).

Erhart, E.; Hollenberg, C.P.: The presence of a defective LEU2 gene on 2µDNA recombinant plasmids of Saccharomyces cerevisiae is responsible for curing and high copy number. J. Bacteriol. 156, 625-635 (1983).

Frederick, K.R.; Tung, J.; Emerick, R.S.; Masiarz, F.R.; Chamberlain, S.H.; Vasavada, A.; Rosenberg, S.; Chakraborty, S.; Schopter, L.M.; Massey, N.: Glucose oxidase from Aspergillus niger. J. Biol. Chem. 265, 3793-3802 (1990).

Hadwick, K.G.; Lewis, M.J.; Semenza, J.; Dean, N.; Pelham, H.R.B.: ERD1, a yeast gene required for the retention of luminal endoplasmic reticulum proteins, affects glycoprotein processing in the Golgi apparatus. EMBO J. 9, 623-630 (1990).

Haselbeck, A.; Hösel, W.: Studies on the effect of the incubation conditions, various detergents and protein concentration on the enzymatic activity of N-glycosidase F (glycopeptidase F) and endoglycosidase F. Topics in Biochemistry 8, 1- 4 (1988).

Hochuli, E.; Doebeli, H.; Schacher, A.: New metal chelate adsorbent selective for proteins and peptides containing neighbouring histidine residues. J. Chromatography 411, 177-184 (1987).

Hochuli, E.; Bannwarth, W.; Doebeli, H.; Genz, R.; Stueber, D.: Genetic approach to facilitate purification of recombinant proteins with a novel metal chelate adsobent. Biotechnology 6, 1321-1325 (1988).

Huffaker, T.C.; Robbins, P.W.: Yeast mutants deficient in protein glycosylation. Proc. Natl. Acad. Sci. 80, 7466-7470 (1983).

Innis, M.A.: Glycosylation of heterologous proteins in Saccharomyces cerevisiae. In: Barr, P.J.; Brake, A.J.; Valenzuela, P. (eds.), Yeast genetic engineering, Butterworths, Stoneham, Mass, pp. 233-246 (1989).

Ito, H.; Jukuda, A.; Murata, K.; Kimura, A.: Transformation of intact yeast cells treated with alkali cations. J. Bacteriol. 153, 163-168 (1983).

Kopetzki, E.; Buckel, P.; Schumacher, G.: Cloning and characterization of baker's yeast alpha glucosidase: overexpression in a yeast strain devoid of vacuolar proteinases. Yeast 5, 11-24 (1989).

Kornfeld, R.; Kornfeld, S.: Assembly of asparagine-linked oligosaccharides. Ann. Rev. Biochem. 54, 631-664 (1985).

Kukuruzinska, M.A.; Bergh, M.L.E.; Jackson, B.J.: Protein glycosylation in yeast. Ann. Rev. Biochem. 56, 915-944 (1987).

Kriechbaum, M.; Heilmann, H,J.; Wientjes, F.J.; Hahn, M.; Jany, K.-D.; Gassen, H.G.; Sharif, F.; Alaeddinoglu, G.: Clonig and DNA sequence analysis of the glucose oxidase gene from Aspergillus niger NRRL-3. FEBS Lett. 255, 63-66 (1989).

Laemmli, U.K.: Cleavage of structural proteins during the assembly of the head of bacteriophage T4. Nature 227, 680-685 (1970).

Maniatis, T. et al., In: Molecular cloning: A laboratory manual. Cold Spring Harbor Laboratory Press, Cold Spring Harbor, New York, (1989).

Moir, D.T.: Yeast mutants with increased secretion efficiency. In: Barr, P.J.; Brake, A.J.; Valenzuela, P. (eds.), Yeast genetic engineering, Butterworths, Stoneham, Mass, pp. 215-231 (1989).

Mullis, K.B.; Faloona, F.A.: Specific synthesis of DNA in vitro via a polymerase-catalyzed chain reaction. Methods Enzymol. 155, 355-350 (1987).

Nakano, A.; Muramatsu, M.: A novel GTP-binding protein, Sarpl, is involved in transport from the endoplasmic reticulum to the Golgi apparatus. J. Cell. Biol. 109, 2677-2691 (1989).

Newman, A.P.; Ferro-Novick, S.: Characterization of new mutants in the early part of the yeast secretory pathway isolated by [3H]mannose suicide selection. J. Cell. Biol. 105, 1587-1594 (1987).

Novick, P.; Field, C.; Schekman, R.: Identification of 23 complementation groups required for post-translational events in the yeast secretory pathway. Cell 21, 205-215 (1980).

Paulson, C.P.: Glycoproteins: what are the sugar chains for? TIBS 14, 272-276 (1989).

Rudolph, H.K., Antebi, A.; Fink, G.R.; Buckley, C.M.; Dorman, T.E.; LeVitre, J.; Davidow, L.S.; Mao, J.; Moir, D.T.: The yeast secretory pathway is perturbed by mutations in PMR1, a member of a Ca2+ ATPase family. Cell 58, 133-145 (1989).

Reddy, V.A.; Johnson, R.S.; Biemann, K.; Williams, R.S.; Ziegler, F.D.; Trimble, R.B.; Maley, F.: Characterization of glycosylation sites in yeast external invertase. I. N-linked oligosaccharide content of the individual sequons. J. Biol. Chem. 263, 6978-6985 (1988).

Runge, K.W.; Robbins, P.W.: Saccharomyces cerevisiae mutants in the early stages of protein glycosylation. In: Bonventre, P.F.; Morello, J.A.M.; Silver, S.D.; Wu, H.C. (eds.), Microbiology-1986. American Society for Microbiology, Washington, D.C. pp. 312-316 (1986).

Schekman, R.; Novick, P.: The secretory process and yeast cell-surface assembly. In: Strathern, J.N.; Jones, E.W.; Broach, J.R. (eds.), The Molecular Biology of the Yeast Saccharomyces, Metabolism and Gene Expression, Cold Spring Harbor Laboratory, New York, pp. 361-398 (1982).

Schmitt, H.D.; Wagner, P.; Pfaff, E.; Gallwitz, D.: The ras-related YPT1 gene product in yeast: a GTP-binding protein that might be involved in microtubule organization. Cell 47, 401-412 (1986).

Schmitt, H.D.; Puzichia, M.; Gallwitz, D.: Study of a temperature-sensitive mutant of the ras-related YPT1 gene product in yeast suggests a role in the regulation of intracellular calcium. Cell 53, 635-647 (1988).

Sherman, F.; Fink, G.R.; Hicks, J.B.: Methods in Yeast Genetics: A Laboratory Manual, Cold Spring Harbor Laboratory, Cold Spring Harbor, New York, (1981).

Tanner, W.; Lehle, L.: Protein glycosylation in yeast. Biochim. Biophys. Acta 906, 81-99 (1987).

Warren, C.E.: Glycosylation- considerations for protein engineering. BFE 7, 392-395 (1990).

Whittington, H.; Kerry-Williams, S.; Bidgood, K.; Dodsworth, N. Peberdy, J.; Dobson, M.; Hinchliffe, E.; Ballance, D.J.: Expression of the Aspergillus niger glucose oxidase gene in A. niger, A. nidulans and Saccharomyces cerevisiae. Curr. Genet. 18, 531-536 (1990).

Zamenhof, S.: Preparation and assay of deoxyribonucleic acid from animal tissue. Methods Enzymol. 3, 696-704 (1957).

Ziegler, F.D.; Maley, F.; Trimble, R.B.: Characterization of glycosylation sites in yeast external invertase. II. Location of the endo-betta-N-acetylglucosaminidase H-resistant sequons. J. Biol. Chem. 263, 6978-6985 (1988).

Duncan, M,J.; Smith, R.A.: Supersecreting mutants of Saccharomyces cerevisiae. EP-A 0 201 208, Collaborative Research Inc.,

Fink, G.R.: Improved supersecreting mutants of Saccharomyces cerevisiae. EP-A 0 382 332, Collaborative Research Inc.,

Kniskern, P.J.; Hagopian, A.; Miller, W.J.; Yamazaki, S.; Ellis, R.W.: Method for producing nonhyperglycosylated hepatitis B virus protein. EP-A 0 344 864, Merck & Co. Inc.,

Kopetzki, E.; Schumacher,G.; Zimmermann, F.K.: Expressionsvektor und Verfahren zur regulierten Herstellung von Proteinen in Eukaryonten. EP-A 0 323 838, Boehringer Mannhein GmbH,

MacKay, V.L.; Welch, S.K.; Yip, C.L.: Methods of regulating protein glycosylation. EP-A 0 314 096, Zymogenetics Inc.

Rosenberg, S.: Production of glucose oxidase in recombinant systems.

## Patentansprüche

1. Hypoglycosylierte rekombinante Glucoseoxidase (GOD) mit einem Molekulargewicht von 68 - 80 kDa, einer spezifischen Aktivität von ca. 200 U/mg Gewichtseinheit, einem Kohlenhydratanteil von ca. 12%, erhältlich durch Expression einer rekombinanten DNA, welche das GOD Gen aus Aspergillus enthält, in den N-glycosylierungsdefekten Hefemutanten DSM 7042, DSM 7338, DSM 7160 oder DSM 7340 oder allelen Mutantenstämmen davon, Fermentation und Isolierung des Enzyms aus dem Kulturüberstand oder den Zellen.

2. Verfahren zur Herstellung einer hypoglycosylierten rekombinanten Glucoseoxidase mit einem Molekulargewicht von ca. 68 - 80 kDa, einer spezifischen Aktivität von ca. 200 U/mg Gewichtseinheit und einem Kohlenhydratanteil von ca. 12% durch Expression einer rekombinanten DNA, welche das GOD Gen enthält, in den N-glycosylierungsdefekten Hefemutanten DSM 7042, DSM 7338, DSM 7160 oder DSM 7340 oder allelen Mutantenstämmen davon, Fermentation und Isolierung des Enzyms aus dem Kulturüberstand oder den Zellen.

3. Verwendung einer Glucoseoxidase nach Anspruch 1 in diagnostischen Tests.

## Claims

1. Hypoglycosylated recombinant glucose oxidase (GOD) with a molecular weight of 68 - 80 kDa, a specific activity of ca. 200 U/mg unit of weight, a carbohydrate portion of ca. 12 % which is obtainable by expression of a recombinant DNA containing the GOD gene from Aspergillus in the N-glycosylation-defective yeast mutants DSM 7042, DSM 7338, DSM 7160 or DSM 7340 or allelic mutant strains thereof, fermentation and isolation of the enzyme from the culture supernatant or the cells.

2. Process for the production of a hypoglycosylated recombinant glucose oxidase with a molecular weight of ca. 68 - 80 kDa, a specific activity of ca. 200 U/mg unit of weight and a carbohydrate portion of ca. 12 % which is obtainable by expression of a recombinant DNA which contains the GOD gene in the N-glycosylation-defective yeast mutants DSM 7042, DSM 7338, DSM 7160 or DSM 7340 or allelic mutant strains thereof, fermentation and isolation of the enzyme from the culture supernatant or the cells.

3. Use of a glucose oxidase as claimed in claim 1 in diagnostic tests.

## Revendications

1. Glucoseoxydase (GOD) recombinante hypoglycosylée, ayant une masse moléculaire de 68-80 kDa, une activité spécifique d'environ 200 U/unité pondérale (mg), une teneur en hydrates de carbone d'environ 12%, qui peut être obtenue par expression d'un ADN recombinant contenant le gène de GOD issu d'Aspergillus, dans les mutants de levure DSM 7042, DSM 7338, DSM 7160 et DSM 7340 dépourvus de N-glycosylation ou dans leurs souches mutantes allèles, fermentation et isolement de l'enzyme à partir du surnageant de culture ou des cellules.

2. Procédé de préparation d'une glucoseoxydase recombinante hypoglycosylée, ayant une masse moléculaire de 68-80 kDa, une activité spécifique d'environ 200 U/unité pondérale (mg), une teneur en hydrates de carbone d'environ 12 %, par expression d'un ADN recombinant contenant le gène de GOD issu d'Aspergillus, dans les mutants de levure DSM 7042, DSM 7338, DSM 7160 et DSM 7340 dépourvus de N-glycosylation ou dans leurs souches mutantes allèles, fermentation et isolement de l'enzyme à partir du surnageant de culture ou des cellules.

3. Utilisation d'une glucoseoxydase selon la revendication 1 dans des analyses de diagnostic.


### SEQUENCE LISTING

(1) GENERAL INFORMATION:
   (i) APPLICANT:
      (A) NAME: BOEHRINGER MANNHEIM GMBH
      (B) STREET: Sandhofer Str. 116
      (C) CITY: Mannheim
      (E) COUNTRY: Germany
      (F) POSTAL CODE (ZIP): D-68305
      (G) TELEPHONE: 0621/759-3197
      (H) TELEFAX: 0621/759-4457
   (ii) TITLE OF INVENTION: Hypoglycosylierte recombinante Glucoseoxidase
   (iii) NUMBER OF SEQUENCES: 10
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
   (vi) PRIOR APPLICATION DATA:
      (A) APPLICATION NUMBER: DE P 42 26 095.7
      (B) FILING DATE: 07-AUG-1992
   (vi) PRIOR APPLICATION DATA:
      (A) APPLICATION NUMBER: DE P 43 01 904.8
      (B) FILING DATE: 07-JAN-1993
(2) INFORMATION FOR SEQ ID NO:1:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:1:
   ATTTCTCCTT ATTGCGCGCT T                                                21
(2) INFORMATION FOR SEQ ID NO:2:
   (i) SEQUENCE CHARACTERISTICS:
      (A) LENGTH: 48 base pairs
      (B) TYPE: nucleic acid
      (C) STRANDEDNESS: single
      (D) TOPOLOGY: linear
   (xi) SEQUENCE DESCRIPTION: SEQ ID NO:2:
   TCTATTCAGC TGTCGACATA GATCTTATGT AATTTAGTTA CGCTTGAC 48
(2) INFORMATION FOR SEQ ID NO:3:
   (i) SEQUENCE CHARACTERISTICS:
      (A) LENGTH: 50 base pairs
      (B) TYPE: nucleic acid
      (C) STRANDEDNESS: single
      (D) TOPOLOGY: linear
   (xi) SEQUENCE DESCRIPTION: SEQ ID NO:3:
   AGATCTATGT CGACAGCTGA ATAGATAAAA TTAGTGCGGA CTTTTTTTTA 50
(2) INFORMATION FOR SEQ ID NO:4:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:4:
   GTCATTTGTA AAGTAAAATT CCAA 24
(2) INFORMATION FOR SEQ ID NO:5:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (xi) SEQUENCE DESCRIPTION: SEQ ID NO:5:
   GCCCGGTACC AGATCTATGC AGACTCTCCT TGTGAGCT 38
(2) INFORMATION FOR SEQ ID NO:6:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:6:
   TCTAGAACTA GTGGATCCCC C 21
(2) INFORMATION FOR SEQ ID NO:7:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (xi) SEQUENCE DESCRIPTION: SEQ ID NO:7:
   GCCGGCGAAC GTGGCGAGAA 20
(2) INFORMATION FOR SEQ ID NO:8:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (xi) SEQUENCE DESCRIPTION: SEQ ID NO:8:
   ATATATCAGC TGTCACTGCA TGCTAGCATA ATCTTCCAAG ATAGC 45
(2) INFORMATION FOR SEQ ID NO:9:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (xi) SEQUENCE DESCRIPTION: SEQ ID NO:9:
   CAGCACCACC ACCACTGACA G 21
(2) INFORMATION FOR SEQ ID NO:10:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (xi) SEQUENCE DESCRIPTION: SEQ ID NO:10:
   CTGTCAGTGG TGGTGGTGCT GCATG                                25